# EUROPÄISCHE PATENTSCHRIFT

(11) **EP 1 762 158 B1**
(45) Veröffentlichungstag und Bekanntmachung des Hinweises auf die Patenterteilung: **04.11.2009**
(21) Anmeldenummer: 06025719.3
(22) Anmeldetag: 05.08.2004
(51) Int. Cl.: A47F 3/04, A61L 9/015

(54) **Gekühltes Warenpräsentationsmöbel mit einem Luftaufbereitungssystem und Verfahren zum Betreiben eines derartigen Warenpräsentationsmöbels**
Refrigerated show case with an air treatment system and method to operate such a show case
Meuble de présentation réfrigéré avec un système pour le traitement d'air et méthode d'opération d'un tel meuble de présentation

(30) Priorität: 19.08.2003 DE 10338096
(43) Veröffentlichungstag der Anmeldung: 14.03.2007
(62) Teilanmeldung aus: 04018593.6
(73) Patentinhaber: Linde Kältetechnik GmbH, 50999 Köln (DE)
(72) Erfinder: Klippel, Gabriele, 55288 Partenheim (DE)
(74) Vertreter: Klunker . Schmitt-Nilson . Hirsch

(56) Entgegenhaltungen:
- EP-A- 0 593 212
- DE-C- 19 616 358
- FR-A- 1 316 616
- FR-A- 1 468 833
- US-A1- 2002 037 240

## Beschreibung

Die Erfindung betrifft ein Warenpräsentationsmöbel, aufweisend wenigstens einen gekühlten Warenraum und wenigstens eine Vorrichtung zur Erzeugung eines in dem Warenpräsentationsmöbel zirkulierenden Kühlluftstromes, der der Kühlung des oder der Warenräume dient.

Ferner betrifft die Erfindung ein Verfahren zum Betreiben eines Warenpräsentationsmöbels.

Unter dem Begriff "Vorrichtung zur Erzeugung eines in dem Warenpräsentationsmöbel zirkulierenden Kühlluftstromes" seien diejenigen Bauteile, wie Verdampfer, Verdichter, Ventilator, etc. zu verstehen, die innerhalb des Warenpräsentationsmöbels zum Zwecke der Erzeugung des zirkulierenden Kühlluftstromes vorgesehen sind. Abhängig davon, ob es sich bei dem jeweiligen Warenpräsentationsmöbel um ein so genanntes steckerfertiges oder ein so genanntes nicht steckerfertiges Möbel handelt, können bestimmte der vorgenannten Bauteile auch entfallen.

Allen bekannten Warenpräsentationsmöbeln ist gemein, dass sich in den Warenräumen, denjenigen Bereichen, die von den zirkulierenden Kühlluftströmen überstrichen werden, sowie auf Bauteilen, wie Verdampfer, etc., Mikroorganismen und Keime bilden und anlagern.

Dies führt dazu, dass die Haltbarkeit bestimmter Lebensmittel verringert wird. Ferner können derartige Mikroorganismen bzw. Keime auch zu einer für den Kunden sowie das Bedienpersonal schädlichen, gesundheitlichen Belastungen - dies gilt insbesondere für Allergiker und Asthmatiker - sowie unangenehmen Geruchsbelästigung führen.

Bisher werden Warenpräsentationsmöbel deshalb in mehr oder weniger regelmäßigen Abständen händisch gereinigt bzw. desinfiziert. Allerdings lassen sich einige Bauteile, wie beispielsweise Verdampfer, und Bereiche nur mit einem unverhältnismäßig grossem Aufwand reinigen, da dazu zunächst Abdeckungen entfernt werden müssten. Aber auch die bei der Reinigung bzw. Desinfizierung verwendeten Reinigungs- bzw. Desinfektionsmittel sind nicht vollständig frei von Komponenten, deren Kontakt mit den präsentierten Waren vermieden werden sollten.

Darüber hinaus ist zu bedenken, dass das händische Reinigen bzw. Desinfizieren von Warenpräsentationsmöbeln zum einen vergleichsweise zeitaufwendig ist und zum anderen nicht sichergestellt werden kann, dass auch wirklich alle Bereiche, bei denen dies erforderlich ist, ausreichend gereinigt bzw. desinfiziert worden sind.

US 2002/0037240 A1 offenbart einen Kühlschrank mit einem Dufterzeuger, der in einem zirkulierenden Kühlluftstrom angeordnet ist, um die Luft in dem Kühlschrank zu deodorieren. Der Dufterzeuger beinhaltet Entladungsmittel, um durch eine Hochspannungsentladung Ozon und UV-Strahlung zu erzeugen, und ein photokatalytisches Modul, um Geruch erzeugende Bestandteile und Keime, die in der Luft enthalten sind, photokatalytisch abzubauen.

Aufgabe der vorliegenden Erfindung ist es, ein gattungsgemässes Warenpräsentationsmöbel sowie ein gattungsgemässes Verfahren zum Betreiben eines Warenpräsentationsmöbels anzugeben, das die vorgenannten Probleme vermeidet, insbesondere das Problem der nur ungenügenden Reinigung schwer zugänglicher Bauteile und Bereiche löst.

Zur Lösung dieser Aufgabe wird ein gattungsgemässes Warenpräsentationsmöbel nach Anspruch 1 sowie ein Verfahren zum Betreiben eines Warenpräsentationsmöbels nach Anspruch 9 vorgeschlagen. Dabei ist der oder den Vorrichtungen zur Erzeugung eines in dem Warenpräsentationsmöbel zirkulierenden Kühlluftstromes wenigstens ein Ozongenerator derart zugeordnet, dass das durch den oder die Ozongeneratoren gebildete Ozon dem oder den zirkulierenden Kühlluftströmen beigemischt wird.

Bei dem erfindungsgemässen Verfahren zum Betreiben eines Warenpräsentationsmöbels wird in Abhängigkeit von dem erfassten Verschmutzungsgrad die Leistung des oder der Ozongeneratoren variiert.

Ozon ist nach Fluor das stärkste, bekannte Oxidationsmittel und vermag sowohl Keime abzutöten als auch Geruchsstoffe zu oxidieren und somit zu neutralisieren.

Ozongeneratoren unterschiedlichster Bauart sind dem Fachmann aus dem Stand der Technik bekannt. Ozongeneratoren neuerer Bauart besitzen eine hohe Lebensdauer und bedürfen darüber hinaus eines nur geringen Wartungsaufwandes.

Innerhalb des Warenpräsentationsmöbels wird wenigstens ein Ozongenerator der oder den Vorrichtungen zur Erzeugung des in dem Warenpräsentationsmöbel zirkulierenden Kühlluftstromes derart zugeordnet, dass das durch ihn gebildete Ozon dem oder den zirkulierenden Kühlluftströmen beigemischt wird. In Abhängigkeit von der Konstruktion des Warenpräsentationsmöbels - insbesondere von dessen Breite - sind unter Umständen mehrere Ozongeneratoren vorzusehen, da mittels lediglich eines Ozongenerators keine gleichmäßig Verteilung des gebildeten Ozons in dem oder den zirkulierenden Kühlluftströmen erreicht werden kann.

Das gebildete und dem oder den zirkulierenden Kühlluftströmen beigemischte Ozon bildet zusammen mit dem oder den zirkulierenden Kühlluftströmen eine "Schutzgasatmosphäre", die die Bildung bzw. Anlagerung von Keimen und Mikroorganismen wirkungsvoll verhindert.

Das mittels des oder der Ozongeneratoren gebildete Ozon wird nunmehr durch den oder die zirkulierenden Kühlluftströme aufgenommen und über diejenigen Flächen und Bereiche des Warenpräsentationsmöbels geführt, die auch mit dem Kühlluftstrom in Kontakt kommen. Die vorgenannten Flächen und Bereiche werden auf diese Weise durch das im Kreislauf geführte Ozon "gereinigt".

Das Warenpräsentationsmöbel weiterbildend wird vorgeschlagen, dass der oder die Ozongeneratoren hinsichtlich ihrer Leistungen variierbar ausgebildet sind.

Entsprechend einer erfindungsgemäßen Ausgestaltung des Warenpräsentationsmöbels, weist dieses Mittel zur Erfassung des Verschmutzungsgrades des- oder derjenigen Bereiche, die mit der zu kühlenden Ware in Kontakt kommen, auf, wobei die Mittel zur Erfassung des Verschmutzungsgrades mit dem oder den Ozongeneratoren in Wirkverbindung stehen.

Insbesondere in der Zusammenschau der beiden vorgenannten Ausgestaltungen des Warenpräsentationsmöbels lässt sich der Ozonisierungsgrad in dem oder den zirkulierenden Kühlluftströmen in Abhängigkeit von dem erfassten Verschmutzungsgrad beeinflussen und variieren. Somit kann die Leistung des oder der Ozongeneratoren dem erfassten Verschmutzungsgrad angepasst werden, was zur Folge hat, dass immer nur die für eine ausreichende Reinigung bzw. Desinfizierung erforderliche Ozonmenge gebildet wird.

Entsprechend einer vorteilhaften Ausgestaltung des erfindungsgemäßen Verfahrens zum Betreiben eines Warenpräsentationsmöbels, wird in regelmäßigen oder unregelmäßigen Abständen die Leistung des oder zumindest eines der Ozongeneratoren erhöht.

Mittels dieser vorteilhaften Ausgestaltung des erfindungsgemäßen Verfahrens zum Betreiben eines Warenpräsentationsmöbels kann in (vor)einstellbaren oder beliebigen zeitlichen Abständen eine intensivere Reinigung des Warenpräsentationsmöbels realisiert werden.

Im Regelfall wird durch den Einsatz eines Ozongenerators täglich im Wesentlichen ununterbrochen eine schützende Gasatmosphäre, die die Bildung von unerwünschten Keimen und Bakterien verhindert, erzeugt. Zusätzlich wird vorzugsweise wenigstens einmal pro Tag eine intensive Reinigung des Warenpräsentationsmöbels realisiert, indem die Leistung des oder zumindest eines der Ozongeneratoren erhöht wird.

In vorteilhafter Weise erfolgt die vorbeschriebene Intensivreinigung in Kombination mit der Abtauphase des oder eines der Verdampfer des erfindungsgemäßen Warenpräsentationsmöbels. Dadurch wird eine Steigerung der Effektivität der Intensivreinigung erreicht, da es zu einer Sekundärreaktion kommt.

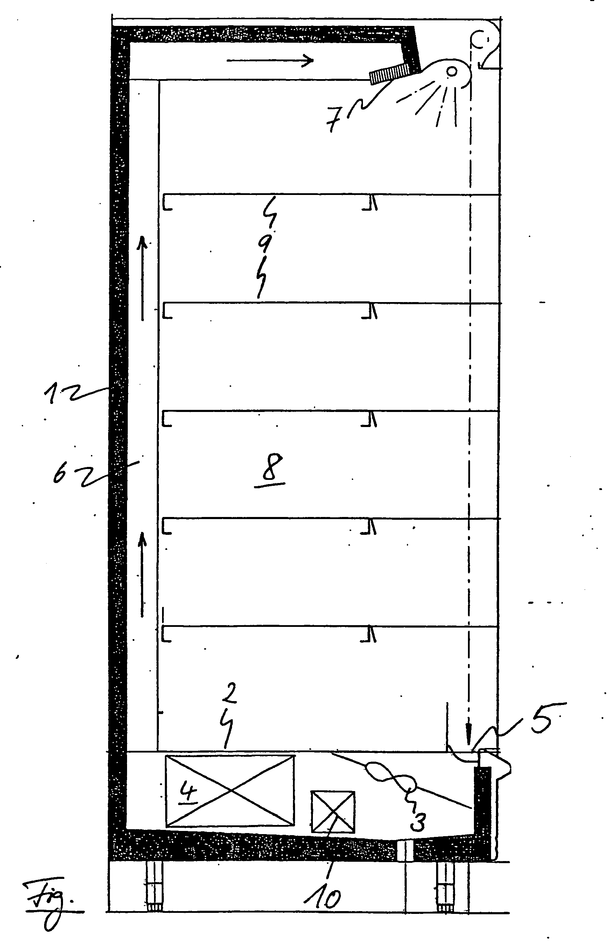

Das erfindungsgemäße Warenpräsentationsmöbel, das erfindungsgemäße Verfahren zum Betreiben eines Warenpräsentationsmöbel sowie weitere Ausgestaltungen desselben seien nachfolgend anhand des in der Figur dargestellten Ausführungsbeispieles näher erläutert. Hierbei zeigt die Figur eine schematisierte, seitliche Schnittdarstellung durch eine mögliche Ausführungsform eines Kühlregales.

Kühlregale weisen einen isolierten Grundkörper 1, der den gekühlten Warenraum 8 umgibt, auf. Innerhalb des gekühlten Warenraumes 8 sind mehrere Warenpräsentationsböden 9 angeordnet.

Im Regelfall unterhalb des untersten Warenpräsentationsbodens 2 sind die für die Erzeugung des bzw. der Kühlluftströme erforderlichen Aggregate angeordnet; in der Figur sind der Übersichtlichkeit halber lediglich ein Ventilator 3 sowie ein Verdampfer bzw. Wärmetauscher 4 dargestellt.

Der über eine im vorderen, unteren Bereich des Kühlregales angeordnete Öffnung 5 eintretende Kühlluftschleier wird mittels des Ventilators 3 durch den Verdampfer bzw. Wärmetauscher 4 geführt und in diesem abgekühlt. Anschließend wird der Kühlluftstrom über den Zuluftkanal 6 zu der im vorderen, oberen Bereich des Kühlregales angeordneten Luftaustrittswabe 7, über die er austritt und entlang der Warenraumöffnung streicht, geführt.

Nunmehr ist wenigstens ein Ozongenerator 10 vorgesehen. Dieser ist - wie in der Figur dargestellt - vorzugsweise im Bereich des Ventilators 3 oder an einem anderen strömungsoptimierten Ort angeordnet, so dass er in bestmöglicher Weise von der in dem Kühlregal zirkulierenden Kühlluft umspült wird bzw. das von ihm gebildete Ozon mit der zirkulierenden Kühlluft in Kontakt kommt. Durch eine derartige Anordnung lässt sich eine gleichmäßige Verteilung des mittels des Ozongenerators 10 gebildeten Ozons in dem zirkulierenden Kühlluftstrom erreichen.

In vorteilhafter Weise sind - wie in der Figur dargestellt - der oder die Ozongeneratoren 10 bei Warenpräsentationsmöbeln, die wenigstens einen Verdampfer oder Wärmetauscher 4 aufweisen, in Strömungsrichtung vor dem Verdampfer oder Wärmetauscher 4 angeordnet.

Diese Anordnung - die eine besonders intensive Reinigung der Verdampfer bzw. Wärmetauscher ermöglicht - ist deshalb von großem Vorteil, da Verdampfer bzw. Wärmetauscher, diejenigen Bauteile bzw. Komponenten innerhalb eines Warenpräsentationsmöbels darstellen, die am stärksten verschmutzen, aber für das Reinigungspersonal praktisch nicht zugänglich sind.

Prinzipiell kann bzw. können die vorgesehenen Ozongeneratoren jedoch auch an anderen geeigneten Stellen innerhalb des Warenpräsentationsmöbels angeordnet werden. Diese Anordnung des bzw. der Ozongeneratoren wird in der Praxis von der Art des jeweiligen Warenpräsentationsmöbels abhängen.

Allgemein gilt, dass - entsprechend einer vorteilhaften Ausgestaltung des erfindungsgemäßen Warenpräsentationsmöbels - der oder die Ozongeneratoren 10 in dem bzw. denjenigen Bereichen, die von dem oder den zirkulierenden Kühlluftströmen am stärksten durch- oder überströmt werden, angeordnet sein sollten. Dadurch wird eine bestmögliche Verteilung des erzeugten Ozons in dem oder den zirkulierenden Kühlluftströmen erreicht, woraus eine optimale Reinigungswirkung resultiert.

Das erfindungsgemäße Warenpräsentationsmöbel weiterbildend wird vorgeschlagen, das Warenpräsentationsmöbel Mittel zur Überwachung des oder der Ozongeneratoren 10 und/oder Mittel zur Überwachung der Mittel zur Erfassung des Verschmutzungsgrades aufweist.

In analoger Weise wird das Verfahren zum Betreiben des erfindungsgemäßen Warenpräsentationsmöbels, wobei das Warenpräsentationsmöbel Mittel zur Überwachung des oder der Ozongeneratoren (10) und/oder Mittel zur Überwachung der Mittel zur Erfassung des Verschmutzungsgrades aufweist, weiterbildend vorgeschlagen, dass die Mittel zur Überwachung in eine lokale und/oder Fernüberwachung des Warenpräsentationsmöbels integriert sind.

Die vorgenannten Ausgestaltungen des erfindungsgemäßen Warenpräsentationsmöbels bzw. des erfindungsgemäßen Verfahrens ermöglichen es nunmehr, den hygienischen Zustand des Warenpräsentationsmöbels bzw. seines Warenraumes lokal und/oder mittels Datenfemübertragung permanent oder in (regelmäßigen) Abständen zu überwachen, entsprechende Nachregelungen vorzunehmen und den jeweiligen Zustand zu dokumentieren. Die hierfür innerhalb des Warenpräsentationsmöbels erforderliche Elektronik und ggf. auch Sensorik kann in der im Regelfall bereits vorhandenen Elektronik bzw. Sensorik integriert werden.

Werden innerhalb eines Supermarktes eine Vielzahl von Warenpräsentationsmöbeln vorgesehen, so wird oftmals ein so genannter Zentralrechner vorgesehen, an den die Regelelektroniken der einzelnen Warenpräsentationsmöbel und damit auch die Mittel zur Überwachung bzw. deren Elektronik angebunden sind.

Mittels der Erfindung wird somit ein Warenpräsentationsmöbel geschaffen, das sich - zumindest in denjenigen Bereichen, die von dem oder den zirkulierenden Kühlluftströmen überstrichen werden - selbst reinigt. Auf den gereinigten Flächen bzw. in den gereinigten Bereichen verbleiben dabei keine Rückstände von Reinigungs- oder Desinfektionsmitteln. Auch wird die Bildung von möglicherweise unangenehmen Gerüchen durch die Erfindung wirkungsvoll verhindert; femer werden vorhandene (unangenehme) Gerüche neutralisiert. Darüber hinaus kann bei einer Vielzahl von Lebensmitteln deren Haltbarkeit verlängert werden.

Des Weiteren wird zudem der Keimgehalt in dem Ladengeschäft bzw. Supermarkt, in dem das erfindungsgemäße Warenpräsentationsmöbel steht, ebenfalls positiv beeinflusst, da zwischen dem zirkulierenden Kühlluftstrom und der das erfindungsgemäße Warenpräsentationsmöbel umgebenden Raumluft ein permanenter Luftaustausch stattfindet.

Es sei betont, dass sich die Erfindung sowohl bei den so genannten steckerfertigen als auch den so genannten nicht steckerfertigen Warenpräsentationsmöbeln realisieren lässt.

## Patentansprüche

1. Warenpräsentationsmöbel, aufweisend wenigstens einen gekühlten Warenraum (8) und wenigstens eine Vorrichtung (3, 4) zur Erzeugung eines in dem Warenpräsentationsmöbel zirkulierenden Kühlluftstromes, der der Kühlung des oder der Warenräume dient, wobei der oder den Vorrichtungen (3, 4) zur Erzeugung eines in dem Warenpräsentationsmöbel zirkulierenden Kühlluftstromes wenigstens ein Ozongenerator (10) derart zugeordnet ist, dass das durch den oder die Ozongeneratoren (10) gebildete Ozon dem oder den zirkulierenden Kühlluftströmen beigemischt wird,
**dadurch gekennzeichnet, dass**
das Warenpräsentationsmöbel Mittel zur Überwachung des oder der Ozongeneratoren (10) aufweist, dass
die Mittel zur Überwachung in eine Fernüberwachung des Warenpräsentationsmöbels integriert sind, und dass
das Warenpräsentationsmöbel Mittel zur Erfassung des Verschmutzungsgrades des- oder derjenigen Bereiche, die mit der zu kühlenden Ware in Kontakt kommen, aufweist und die Mittel zur Erfassung des Verschmutzungsgrades mit dem oder den Ozongeneratoren (10) in Wirkverbindung stehen

2. Warenpräsentationsmöbel nach Anspruch 1, **dadurch gekennzeichnet, dass** die Mittel zur Fernüberwachung Mittel zur Datenfernübertragung aufweisen.

3. Warenpräsentationsmöbei nach einem der vorhergehenden Ansprüche,
**dadurch gekennzeichnet, dass** die Mittel zur Überwachung an einen Zentralrechner angeschlossen sind.

4. Warenpräsentationsmöbel nach einem der vorhergehenden Ansprüche, **dadurch gekennzeichnet, dass** der oder die Ozongeneratoren (10) hinsichtlich ihrer Leistungen variierbar ausgebildet sind.

5. Warenpräsentationsmöbel nach einem der vorhergehenden Ansprüche, **dadurch gekennzeichnet, dass** das Warenpräsentationsmöbel wenigstens einen Ventilator (3) aufweist und dass der oder die Ozongeneratoren (10) in unmittelbarer Nähe zum dem oder einem der Ventilatoren (3) angeordnet sind.

6. Warenpräsentationsmöbel nach einem der vorhergehenden Ansprüche, wobei das Warenpräsentationsmöbel wenigstens einen Verdampfer oder Wärmetauscher (4) aufweist, **dadurch gekennzeichnet, dass** der oder die Ozongeneratoren (10) in Strömungsrichtung vor dem Verdampfer oder Wärmetauscher (4) angeordnet sind.

7. Warenpräsentationsmöbel nach einem der vorhergehenden Ansprüche, **dadurch gekennzeichnet, dass** der oder die Ozongeneratoren (10) in dem bzw. denjenigen Bereichen, die von dem oder den zirkulierenden Kühlluftströmen am stärksten durch- oder überströmt werden, angeordnet sind.

8. Warenpräsentationsmöbel nach einem der vorhergehenden Ansprüche, **dadurch gekennzeichnet, dass** das Warenpräsentationsmöbel Mittel zur Überwachung der Mittel zur Erfassung des Verschmutzungsgrades aufweist.

9. Verfahren zum Betreiben eines Warenpräsentationsmöbels nach einem der vorhergehenden Ansprüche, **dadurch gekennzeichnet, dass** der hygienische Zustand des Warenpräsentationsmöbels mittels Fernüberwachung überwacht wird.

10. Verfahren nach Anspruch 9, **dadurch gekennzeichnet, dass** die Leistung des oder der Ozongeneratoren (10) in Abhängigkeit von dem erfassten Verschmutzungsgrad variiert wird.

11. Verfahren nach Anspruch 9 oder 10, **dadurch gekennzeichnet, dass** der jeweilige hygienische Zustand dokumentiert wird.

12. Verfahren nach einem der Ansprüche 9 bis 11, **dadurch gekennzeichnet, dass** in regelmässigen oder unregelmässigen Abständen die Leistung des oder zumindest eines der Ozongeneratoren (10) erhöht wird.

13. Verfahren nach Anspruch 12, wobei der oder zumindest einer der Verdampfer oder Wärmetauscher des Warenpräsentationsmöbels in regelmässigen oder unregelmässigen Abständen einem Abtauprozess unterworfen wird, **dadurch gekennzeichnet, dass** die in regelmässigen oder unregelmässigen Abständen erfolgende Erhöhung der Leistung des oder zumindest eines der Ozongeneratoren (10) im Wesentlichen zeitgleich mit dem oder einem Abtauprozess erfolgt.

## Claims

1. A goods presentation furniture, comprising at least one refrigerated goods space (8) and at least one means (3, 4) for generating a refrigerating air flow circulating within the goods presentation furniture and refrigerating the goods space(s), wherein at least one ozone generator (10) is associated with the means (3, 4) for generating a refrigerating air flow circulating within the goods presentation furniture such that ozone formed by the ozone generator(s) (10) is added to the circulating refrigerating air flow(s),
**characterized in that**
the goods presentation furniture comprises means for monitoring the ozone generator(s) (10), and
the means for monitoring are integrated in a remote monitoring unit of the goods presentation furniture, and that
the goods presentation furniture comprises means for detecting the degree of pollution of the portion(s) coming into contact with the goods to be refrigerated and the means for detecting the degree of pollution is/are in functional interaction with the ozone generator(s) (10).

2. A goods presentation furniture according to claim 1,
**characterized in that** the means for remote monitoring comprise means for remote data transfer.

3. A goods presentation furniture according to any of the preceding claims,
**characterized in that** the means for monitoring are connected to a central computer.

4. A goods presentation furniture according to any of the preceding claims,
**characterized in that** the ozone generator(s) (10) is (are) variable as regards the output thereof.

5. A goods presentation furniture according to any of the preceding claims,
**characterized in that** the goods presentation furniture comprises at least one fan (3) and that the ozone generator(s) (10) is (are) arranged in immediate proximity to the or one of the fans (3).

6. A goods presentation furniture according to any of the preceding claims,
with the goods presentation furniture comprising at least one evaporator or heat exchanger (4), **characterized in that** the ozone generator(s) (10) is (are) arranged upstream of the evaporator or heat exchanger (4) in the direction of flow.

7. A goods presentation furniture according to any of the preceding claims,
**characterized in that** the ozone generator(s) (10) is (are) arranged in the portion(s) in which the flow of the circulating refrigerating air through or across the same is strongest.

8. A goods presentation furniture according to any of the preceding claims,
**characterized in that** the goods presentation furniture comprises means for monitoring the means for detecting the degree of pollution.

9. A method of operating a goods presentation furniture according to any of the preceding claims,
**characterized in that** the hygienic state of the goods presentation furniture is monitored by remote monitoring.

10. A method according to claim 9,
**characterized in that** the output of the ozone generator(s) (10) is varied in accordance with the degree of pollution detected.

11. A method according to claim 9 or 10,
**characterized in that** the respective hygienic state is documented.

12. A method according to any of claims 9 to 11,
**characterized in that** the output of the or at least one of the ozone generators (10) is increased in regular or irregular intervals.

13. A method according to claim 12,
in which the or at least one of the evaporators or heat exchangers of the goods presentation furniture is subjected to a defrosting process in regular or irregular intervals,
**characterized in that** the increase in output of the or at least one of the ozone generator(s) (10) in regular or irregular intervals is effected substantially at the same time as the or a defrosting process.

## Revendications

1. Meuble présentoir pour produits, présentant au moins un compartiment de produits réfrigéré (8) et au moins un dispositif (3, 4) pour créer un flux de circulation d'air de refroidissement dans le meuble présentoir pour produits, qui sert au refroidissement du ou des compartiments, un générateur d'ozone (10) étant affecté au ou aux dispositifs (3, 4) pour créer une circulation d'air de refroidissement dans le meuble présentoir pour produits, de telle manière que l'ozone produite par le ou les générateurs d'ozone soit mélangé au ou aux flux d'air de refroidissement qui circule(nt),
**caractérisé en ce que**
le meuble présentoir pour produits présente des moyens de surveillance du ou des générateurs d'ozone(10), que
les moyens de surveillance sont intégrés dans une télésurveillance du meuble présentoir pour produits, et **en ce que**
le meuble présentoir pour produits présente des moyens pour détecter le degré d'encrassement de la ou des zones venant en contact avec le produis à réfrigérer et les moyens pour détecter le degré d'encrassement sont en liaison effective avec le ou les générateurs d'ozone.

2. Meuble présentoir pour produits selon la revendication, **caractérisé en ce que** les moyens de télésurveillance comprennent des moyens de télétransmission de données.

3. Meuble présentoir pour produits selon l'une des revendications précédentes, **caractérisé en ce que** les moyens de surveillance sont reliés à un ordinateur central.

4. Meuble présentoir pour produits selon l'une des revendications précédentes, **caractérisé en ce que** le ou les générateurs d'ozone(10) sont réalisés de façon variable quant à leurs puissances.

5. Meuble présentoir pour produits selon l'une des revendications précédentes, **caractérisé en ce que** le meuble présentoir pour produits présente au moins un ventilateur et **en ce que** le ou les générateurs d'ozone (10) sont disposés à proximité immédiate du ou de l'un des ventilateurs.

6. Meuble présentoir pour produits selon l'une des revendications précédentes, le meuble présentoir pour produits présentant au moins un évaporateur ou échangeur de chaleur (4), **caractérisé en ce que** le ou les générateurs d'ozone(10) sont disposé en amont de l'évaporateur ou échangeur de chaleur (4) dans le sens de l'écoulement.

7. Meuble présentoir pour produits selon l'une des revendications précédentes, **caractérisé en ce que** le ou les générateurs d'ozone(10) sont disposés dans la ou les zones qui sont parcourues ou submergés le plus fortement par le ou les flux d'air de refroidissement circulant.

8. Meuble présentoir pour produits selon l'une des revendications précédentes, **caractérisé en ce que** le meuble présentoir pour produits présente des moyens de surveillance des produits pour détecter le degré d'encrassement.

9. Procédé de mise en oeuvre d'un meuble présentoir pour produits selon l'une des revendications précédentes, **caractérisé en ce que** l'état d'hygiène du meuble présentoir pour produits est surveillé par une télésurveillance.

10. Procédé selon la revendication 9, **caractérisé en ce que** la puissance du ou des générateurs d'ozone (10) varie en fonction du degré d'encrassement détecté.

11. Procédé selon la revendication 9 ou 10, **caractérisé en ce que** l'état d'hygiène respectif est documenté.

12. Procédé selon l'une des revendications 9 à 11, **caractérisé en ce que** la puissance du ou de l'un des générateurs d'ozone (10) s'élève par palier réguliers ou irréguliers.

13. Procédé selon la revendication 12, le ou au moins l'un des évaporateurs ou échangeurs de chaleur du meuble présentoir pour produits étant soumis par palier réguliers ou irréguliers à un processus de dégivrage, **caractérisé en ce que** l'augmentation de la puissance par palier réguliers ou irréguliers du ou de l'un des générateurs d'ozone (10) s'effectue essentiellement en même temps avec le ou un processus de dégivrage.
